(19) Europäisches Patentamt
European Patent Office
Office européen des brevets

(11) Veröffentlichungsnummer: **0 074 539**
**A1**

# (12) EUROPÄISCHE PATENTANMELDUNG

(21) Anmeldenummer: 82107875.5

(22) Anmeldetag: 27.08.82

(51) Int. Cl.³: C 07 D 253/06
A 01 N 43/64
//C07C121/34

(30) Priorität: 07.09.81 DE 3135413

(43) Veröffentlichungstag der Anmeldung: 23.03.83 Patentblatt 83/12

(84) Benannte Vertragsstaaten: AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Kranz, Eckart, Dr.
Am Acker 9
D-5600 Wuppertal 1(DE)

(72) Erfinder: Findeisen, Kurt, Dr.
In der Follmühle 10
D-5068 Odenthal(DE)

(72) Erfinder: Eue, Ludwig, Dr.
Paul-Klee-Strasse 36
D-5090 Leverkusen(DE)

(72) Erfinder: Schmidt, Robert R., Dr.
Im Waldwinkel 110
D-5068 Bergisch-Gladbach 2(DE)

## (54) Azomethine von 4-Amino-6-halogen-tert.-butyl-1,2,4-triazin-5-onen, Verfahren zu ihrer Herstellung sowie ihre Verwendung als Herbizide.

(57) Neue Azomethine von 4-Amino-6-halogen-tert.-butyl-1,2,4-triazin-5-onen der allgemeinen Formel

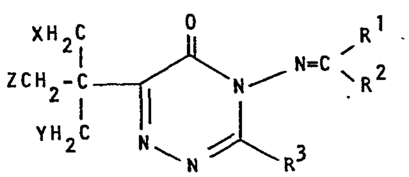

(1)

in welcher
$R^1$ für Wasserstoff oder Alkyl steht,
$R^2$ für Alkyl, Cycloalkyl, Cycloalkenyl oder für gegebenenfalls substituiertes Phenyl steht,
$R^3$ für Alkylthio, Alkylamino oder Dialkylamino steht,
X für Halogen steht und
Y und Z für Wasserstoff oder Halogen stehen,
ein Verfahren zu ihrer Herstellung und ihre Verwendung als Herbizide.

EP 0 074 539 A1

0074539

BAYER AKTIENGESLLSCHAFT
Zentralbereich
Patente, Marken und Lizenzen
5090 Leverkusen, Bayerwerk
Bi/Fr
Ia

04. 09. 81

# Azomethine von 4-Amino-6-halogen-tert.-butyl-1,2,4-triazin-5-onen, Verfahren zu ihrer Herstellung sowie ihre Verwendung als Herbizide.

Die vorliegende Erfindung betrifft neue Azomethine von 4-Amino-6-halogen-tert.-butyl-1,2,4-triazin-5-onen, ein Verfahren zu ihrer Herstellung sowie ihre Verwendung als Herbizide, insbesondere als selektive Herbizide.

Es ist bereits bekannt geworden, daß Azomethine von 3-Alkyl-4-amino-6-tert.-butyl-1,2,4-triazin-5-onen, wie z.B. 3-Ethyl-4-cyclohexylidenamino-6-tert.-butyl-1,2,4-triazin-5-on, als Herbizide verwendet werden können (vgl. DE-OS 22 38 206 [LeA 14 483]). In bestimmten Kulturen ist jedoch ein selektiver Einsatz der vorbekannten Azomethine nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Azomethinen ist demnach bei verschiedenen Nutzpflanzen nicht ausreichend.

Es wurden nun Azomethine von 4-Amino-6-halogen-tert.-butyl-1,2,4-triazin-5-onen der allgemeinen Formel I

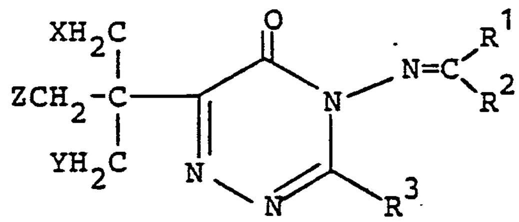

(I)

in welcher

$R^1$ für Wasserstoff oder Alkyl steht,

$R^2$ für Alkyl, Cycloalkyl, Cycloalkenyl oder für gegebenenfalls substituiertes Phenyl steht,

$R^3$ für Alkylthio, Alkylamino oder Dialkylamino steht,

X für Halogen steht und

Y und Z für Wasserstoff oder Halogen stehen,

aufgefunden; diese Verbindungen sind neu.

Weiterhin wurde gefunden, daß man die Azomethine von 4-Amino-6-halogen-tert.-butyl-1,2,4-triazin-5-onen der Formel (I) erhält, wenn man 4-Amino-6-halogen-tert.-butyl-1,2,4-triazin-5-one der allgemeinen Formel II

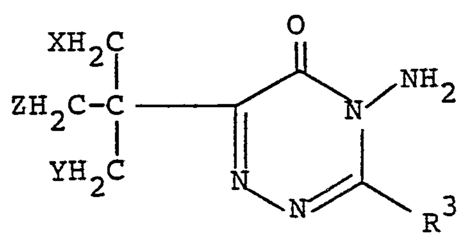

(II)

in welcher

$R^3$, X, Y und Z die oben angegebene Bedeutung haben,

mit Carbonylverbindungen der Formel III

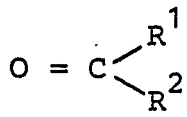

(III)

in welcher

$R^1$ und $R^2$ die oben angegebene Bedeutung haben,

gegebenenfalls in Gegenwart eines Verdünnungsmittels und gegebenenfalls in Gegenwart eines basischen oder sauren Katalysators umsetzt; die Carbonylverbindungen der Formel (III) können dabei gegebenenfalls in Form von Acetalen bzw. Ketalen eingesetzt werden.

Außerdem wurde gefunden, daß die Azomethine von 4-Amino-6-halogen-tert.-butyl-1,2,4-triazin-5-onen der Formel (I) gute herbizide, insbesondere selektiv-herbizide Eigenschaften aufweisen.

Ueberraschenderweise zeigen die erfindungsgemäßen Verbindungen gegenüber dem bekannten 3-Ethyl-4-cyclohexylidenamino-6-tert.-butyl-1,2,4-triazin-5-on, welches chemisch und wirkungsmäßig eine naheliegende Verbindung ist, neben einer besseren allgemeinen herbiziden Wirkung, insbesondere eine bessere Verträglichkeit bei wichtigen Kulturpflanzen, wie bei Mais, Sojabohnen und Baumwolle. Die erfindungsgemäßen Wirkstoffe stellen somit eine Bereicherung der herbiziden Mittel, insbesondere der selektiven chemischen Unkrautbekämpfung dar.

Die erfindungsgemäßen Azomethine sind durch die Formel (I) allgemein definiert. In dieser Formel stehen vorzugsweise

$R^1$ für Wasserstoff oder geradkettiges und verzweigtes Alkyl mit 1 bis 6 Kohlenstoffatomen;

$R^2$ für geradkettiges oder verzweigtes Alkyl mit 1 bis 6 Kohlenstoffatomen, Cycloalkyl mit 3 bis 6 Kohlenstoffatomen, Cycloalkenyl mit 5 bis 6 Kohlenstoffatomen oder für gegebenenfalls substituiertes Phenyl, wobei als Substituenten beispielsweise genannt seien: Halogen, Alkyl mit 1 bis 4 Kohlenstoffatomen, Halogenalkyl mit 1 bis 2 Kohlenstoff- und bis zu 5 gleichen oder verschiedenen Halogenatomen (wie vorzugsweise Fluor- und Chloratomen), Alkoxy und Alkylthio mit jeweils 1 bis 2 Kohlenstoffatomen, Cyano und Nitro;

$R^3$ für geradkettiges oder verzweigtes Alkylthio mit 1 bis 4 Kohlenstoffatomen, sowie für Alkylamino und Dialkylamino mit jeweils 1 bis 6 Kohlenstoffatomen in jedem Alkylteil;

X für Halogen steht und

Y und Z für Wasserstoff oder Halogen stehen.

Besonders bevorzugt sind diejenigen Verbindungen der Formel (I), in denen

$R^1$ für Wasserstoff oder geradkettiges und verzweigtes Alkyl mit 1 bis 4 Kohlenstoffatomen steht;

$R^2$ für geradkettiges oder verzweigtes Alkyl mit 1 bis 4 Kohlenstoffatomen, Cyclohexyl oder Cyclohexenyl oder für gegebenenfalls substituiertes Phenyl steht, wobei als Substituenten genannt seien: Fluor, Chlor, Methyl, Ethyl und Nitro;

$R^3$ für Methyl-, Ethyl- oder Propylthio, ferner für Methyl-, Ethyl-, Propyl-, Hexyl-, Dimethyl-, Diethyl- sowie Ethylmethylamino steht;

X für Fluor, Chlor oder Brom steht sowie

Y und Z für Wasserstoff, Fluor, Chlor oder Brom stehen.

Verwendet man beispielsweise 4-Amino-6-chlor-tert.-butyl-3-methylthio-1,2,4-triazin-5-on und Aceton als Ausgangsstoffe, so kann der Reaktionsablauf durch das folgende Formelschema wiedergegeben werden:

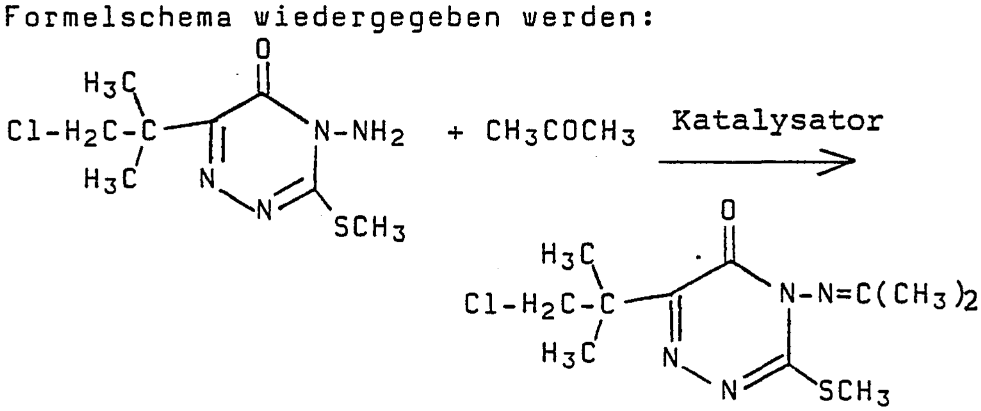


Die für das erfindungsgemäße Verfahren als Ausgangsstoffe zu verwendenden 4-Amino-6-halogen-tert.-butyl-1,2,4-triazin-5-one sind durch die Formel (II) allgemein definiert. In dieser Formel stehen $R^3$, X,Y und Z vorzugsweise für die Reste, die bereits im Zusammenhang mit der Beschreibung der erfindungsgemäßen Stoffe der Formel (I) vorzugsweise für diese Substituenten genannt wurden.

Die 4-Amino-6-halogen-tert.-butyl-1,2,4-triazin-5-one der Formel (II) sind noch nicht bekannt; sie sind jedoch Gegenstand einer eigenen älteren, nicht vorveröffentlichten Patentanmeldung (vgl. Deutsche Patentanmeldung P 30 37 300 vom 2.10.1980 [Le A 20 631]) und können hergestellt werden, indem man in einer ersten Stufe Halogenpivaloylcyanide der Formel IV

$$ZCH_2 - \underset{\displaystyle CH_2Y}{\overset{\displaystyle CH_2X}{\underset{|}{\overset{|}{C}}}} - CO - CN \qquad \text{(IV)}$$

in welcher

X,Y und Z die oben angegebene Bedeutung haben,

gegebenenfalls in Gegenwart einer unter den Reaktionsbedingungen flüssigen, niederaliphatischen Carbonsäure als Lösungsmittel, wie z.B. Essigsäure, mit einer starken anorganischen Säure, wie z.B. Halogenwasserstoffsäuren, bei Temperaturen zwischen -20 und + 5o°C umsetzt; und die hierbei entstehenden halogenierten Trimethylbrenztraubensäureamide der Formel Va

$$ZCH_2 - \underset{\displaystyle CH_2Y}{\overset{\displaystyle CH_2X}{\underset{|}{\overset{|}{C}}}} - CO - CO - NH_2 \qquad \text{(Va)}$$

in welcher

X,Y und Z die oben angegebene Bedeutung haben,

in einer zweiten Stufe in an sich bekannter Weise entweder direkt in der anfallenden Lösung oder nach Zwischenisolierung, gegebenenfalls nach vorheriger Verseifung zu den freien halogenierten Trimethylbrenztraubensäuren der Formel Vb

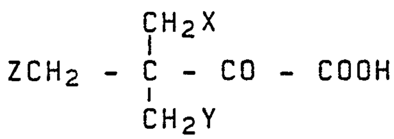 (Vb)

in welcher

X, Y und Z die oben angegebene Bedeutung haben,

mit Thiocarbohydrazid der Formel VI

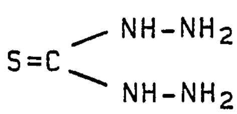 (VI)

in wässriger bzw. in wässrig-saurer Lösung, wie z.B. in salzsaurer Lösung, gegebenenfalls in Gegenwart eines organischen Verdünnungsmittels, wie Dimethylformamid, bei Temperaturen zwischen 0 und 100 °C zu 4-Amino-6-halogen-tert.-butyl-3-mercapto-1,2,4-triazin-5-onen bzw. den isomeren 3-Thioxo-Verbindungen der Formel VII

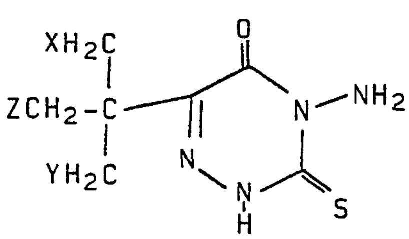 (VII)

in welcher

X, Y und Z die oben angegebene Bedeutung haben,

umsetzt; diese in einer dritten Stufe in an sich bekannter Weise in alkoholischer Lösung mittels Alkylhalogenid, wie z.B. Methyljodid oder -bromid, zu 4-Amino-6-halogen-tert.-butyl-3-alkylthio-1,2,4-triazin-5-onen der Formel IIa

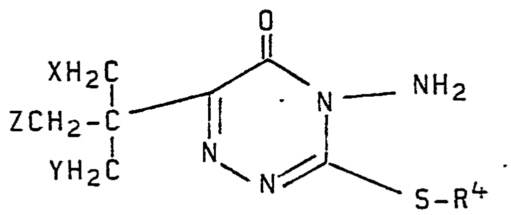

(IIa)

in welcher

X,Y und Z die oben angegebene Bedeutung haben und

$R^4$ für Alkyl, vorzugsweise mit 1 bis4 Kohlenstoffatomen, steht,

bei Temperaturen zwischen 0 und 100°C alkyliert und diese gegebenenfalls in einer vierten Stufe mit Aminen der Formel VIII

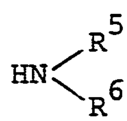

(VIII)

in welcher

$R^5$ für Wasserstoff oder Alkyl, vorzugsweise mit 1 bis 6 Kohlenstoffatomen, steht, und

$R^6$ für Alkyl, vorzugsweise mit 1 bis 6 Kohlenstoffatomen, steht,

in Gegenwart eines organischen Lösungsmittels, wie insbesondere Isopropanol/Eisessig, bei Temperaturen zwischen 40 ind 150°C umsetzt (vgl. hierzu DE-OS 30 03 541 und DE-OS 30 03 542).

Die Halogenpivaloylcyanide der Formel (IV) sind noch nicht bekannt, auch sie sind Gegenstand der o.g. eigenen älteren, nicht vorveröffentlichten Anmeldung (vgl. Deutsche Patentanmeldung P 30 37 300 vom 2.10.1980 [Le A 20 631]). Sie werden erhalten, indem man die entsprechenden Halogenpivaloylhalogenide bzw. -anhydride der Formeln IXa bzw. IXb.

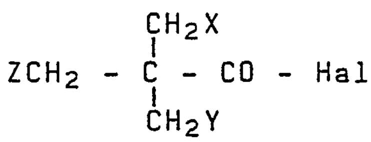

(IXa)

bzw.

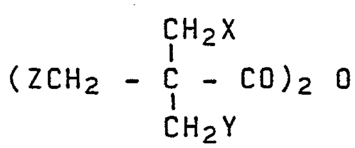

(IXb)

in welchen

Hal für Halogen, vorzugsweise Chlor oder Brom, steht und

X,Y und Z die oben angegebenen Bedeutungen haben,

mit Trimethylsilylcyanid gegebenenfalls in Gegenwart eines inerten organischen Lösungsmittels, bei Temperaturen zwischen 80 und 180°C umsetzt. Trinethylsilylcyanid, $(CH_3)_3Si-CN$, ist bekannt (vgl. z.B. Synthesis 1979, Seiten 522 und 523).

Die Halogenpivaloylhalogenide bzw. -anhydride der Formeln (IXa) und (IXb) sind bekannt oder können nach bekannten Verfahren hergestellt werden.

Die außerdem für das erfindungsgemäße Verfahren als Ausgangsstoffe zu verwendenden Carbonylverbindungen sind durch die Formel (III) allgemein definiert. In dieser Formel stehen $R^1$ und $R^2$ vorzugsweise für die Reste, die bereits im Zusammenhang mit der Beschreibung der erfindungsgemäßen Stoffe der Formel (I) vorzugsweise für diese Substituenten genannt wurden.

Als Beispiele für Carbonylverbindungen der Formel (III) seien genannt: Propionaldehyd, Isobutyraldehyd, Benzaldehyd, Tetrahydro- und Hexahydrobenzaldehyd, Aceton, Methylethylketon, Methylisopropylketon.

Die Carbonylverbindungen der Formel (III), d.h. Aldehyde und Ketone, sind allgemein bekannte Verbindungen der organischen Chemie.

Als Verdünnungsmittel kommen für das erfindungsgemäße Verfahren alle inerten organischen Lösungsmittel infrage. Hierzu gehören vorzugsweise Alkohole, wie Methanol, Ethanol und tert.-Butanol; Ether, wie insbesondere Tetrahydrofuran und Dioxan; halogenierte Kohlenwasserstoffe, wie Methylenchlorid; sowie aromatische Kohlenwasserstoffe, wie Benzol, Toluol oder Xylol.

Das erfindungsgemäße Verfahren wird gegebenenfalls in Gegenwart eines basischen oder eines sauren Katalysators durchgeführt. Hierbei können alle üblichen basischen Katalysatoren eingesetzt werden, wie z.B. Dimethylbenzylamin. Als saure Katalysatoren kommen insbesondere p-Toluolsulfonsäure oder saure Kationenaustauscher in Betracht.

Die Reaktionstemperaturen können bei der Durchführung des erfindungsgemäeßn Verfahrens in einem größeren Bereich variiert werden. Im allgemeinen arbeitet man bei Temperaturen zwischen -50 und +150°C, vorzugsweise zwischen -10 und +120°C.

Bei der Durchführung des erfindungsgemäßen Verfahrens setzt man auf 1 Mol 1,2,4-Triazin-5-on der Formel (II) 1 bis 2 Mol Carbonylverbindung der Formel (III) und gegebenenfalls 0,01 bis 0,1 Mol Katalysator ein. Die Isolierung der Verbindungen der Formel (I) erfolgt in üblicher Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z,B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum,Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaenum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuss-, Bananen-, Kaffee-, Tee-, Gummi-, Oelpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Kulturpflanzen, wie z.B. in Mais, Soja und Baumwolle zu bekämpfen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.
Diese Formulierungen werden in bekannter Weise hergestellt, z.B. durch Vermischen der Wirkstoffe mit Streckmitteln,

also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen

Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Eumulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die angewandte Wirkstoffmenge kann in einem größeren Bereich schwanken. Sie hängt im wesentlichen ab von der Art des gewünschten Effektes. Im allgemeinen liegen die Aufwandmengen zwischen 0,1 und 10kg Wirkstoff pro Hektar Bodenfläche, vorzugsweise zwischen 0,1 und 5 kg pro ha.

In entsprechenden Aufwandmengen zeigen die erfindungsgemäßen Verbindungen auch fungizide Wirksamkeit, insbesondere gegen Getreidekrankheiten.

Die nachfolgenden Beispiele dienen zur weiteren Erläuterung der Erfindung.

Herstellungsbeispiele

Beispiel 1

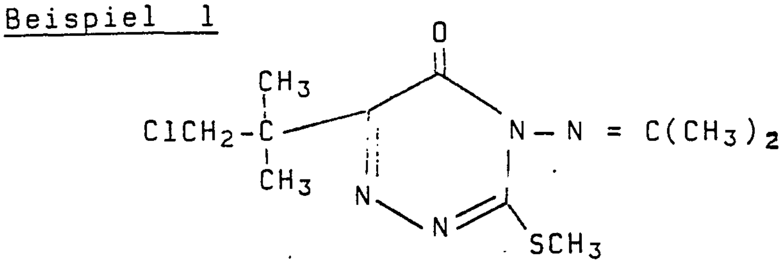

(1)

Zu 12,4g (0,05 Mol) 4-Amino-6-chlor-tert.-butyl-3-methylthio-1,2,4-triazin-5-on und 130 ml Aceton werden bei Raumtemperatur 0,5g p-Toluolsulfonsäure gegeben. Die Reaktionslösung wird 15 Stunden unter Rückfluß und Rühren erhitzt. Danach läßt man abkühlen, engt ein, nimmt den Rückstand in Essigester auf und wäscht dreimal mit je 100 ml Wasser. Nach Trennung über eine Kieselgelsäule (Kieselgel 60; Fließmittel Ether/Petrolether 2:1) erhält man 2,7g (18,7 % der Theorie) 6-Chlor-tert.-butyl-4-isopropylidenamino-3-methylthio-1,2,4-triazin-5-on vom Brechungsindex $n_D^{20}$ =1,557.

Herstellung des Ausgangsproduktes

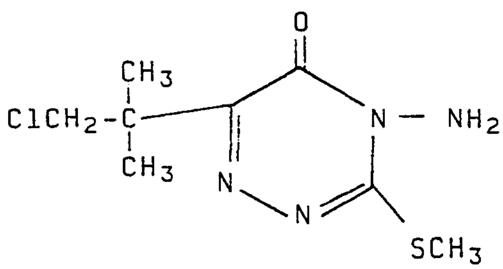

Zu 9 Liter einer Lösung von Bromwasserstoff in Eisessig (33%-ig) werden unter Rühren 2,31 kg(15,88 Mol) ß-Chlorpivaloylcyanid bei Raumtemperatur gegeben. Man läßt 4 Stunden bei Raumtemperatur nachrühren. Anschließend werden bei 7 bis 10 °C 288 ml (15,88 Mol) Wasser zugegeben (exotherme Reaktion, ca. 37°C) und 3 Stunden bei Raumtemperatur nachgerührt. Die Reaktionslösung wird danach

bei 7 bis 10 °C in eine Mischung von 2,03 kg Thiocarbohydrazid und 15,9 Liter 1n Salzsäure eingetragen (stark exotherme Reaktion). Dieses Reaktionsgemisch wird 2 Stunden bei 7 bis 10°C und 14 Stunden bei Raumtemperatur nachgerührt. Danach wird das ausfallende Kristallisat abfiltriert, mit Wasser gewaschen und getrocknet. Man erhält 2995 g (80,4 % der Theorie) rohes 4-Amino-6-chlor-tert.-butyl-3-mercapto-1,2,4-triazin-5-on vom Schmelzpunkt 202-208°C.

2813,4 g (12 Mol) des so erhaltenen 4-Amino-6-chlor-tert.-butyl-3-mercapto-1,2,4-triazin-5-ons werden in 15 Liter 1n Natronlauge gelöst. Nach vollständiger Lösung des Produktes werden bei 7 bis 10 °C 1873,6 g Methyljodid zugetropft. Nach beendeter Zugabe läßt man 2 Stunden bei 7 bis 10°C und über Nacht bei Raumtemperatur nachrühren. Danach wird der entstandene Feststoff abgesaugt, mit Wasser gewaschen und getrocknet. Man erhält 1938g (65 % der Theorie) 4-Amino-6-chlor-tert.-butyl-3-methylthio-1,2,4-triazin-5-on vom Schmelzpunkt 98°C.

Herstellung von ß-Chlorpivaloylcyanid:

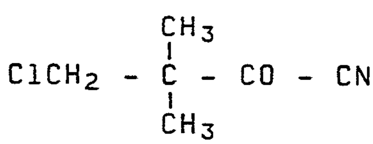

8300g (49,5 Mol) 92,5 %-iges ß-Chlorpivaloylchlorid werden auf 100 °C erwärmt und innerhalb von etwa 2 Stunden mit 4950g (50 Mol) Trimethylsilylcyanid versetzt. Das entstehende Trimethylsilylchlorid wird gleichzeitig abdestilliert. Nach beendeter Zugabe wird die Temperatur langsam auf 140°C gesteigert und das Reaktionsgemisch etwa 1,5 Stunden bei dieser Temperatur gerührt. Anschließend wird die Reaktionsmischung im Vakuum destilliert. Man erhält 7500 g ß-Chlorpivaloylcyanid vom Siedepunkt 62-65°C/16 mbar.

Beispiel 2

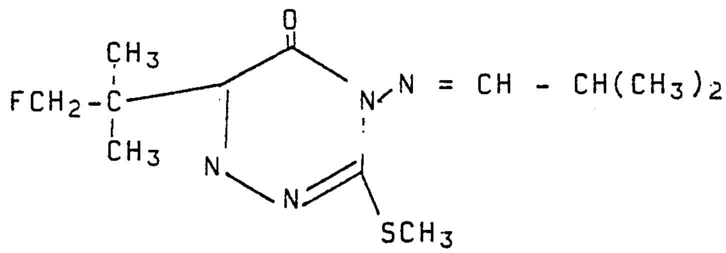

(2)

Zu 0,5 g p-Toluolsulfonsäure in 150 ml Toluol werden 23,2 g (0,1 Mol) 4-Amino-6-fluor-tert.-butyl-3-methylthio-1,2,4-triazin-5-on und 8,64 g (0,12 Mol) Isobutyraldehyd gegeben. Die Reaktionslösung wird 3 Stunden am Wasserabscheider unter Rückfluß erhitzt. Man läßt abkühlen, engt ein und kristallisiert den Rückstand aus Essigester/Petrolether um. Man erhält 13,4 g (47 % der Theorie) 6-Fluor-tert.-butyl-4-isobutylidenamino-3-methylthio-1,2,4-triazin-5-on vom Schmelzpunkt 134-37°C.

Herstellung des Ausgangsproduktes

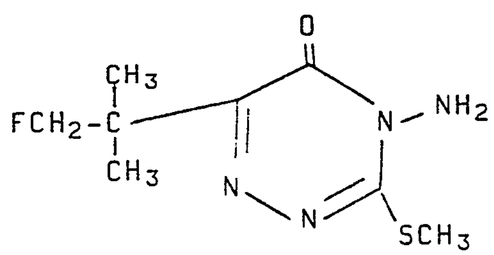

Entsprechend Beispiel 1 erhält man, ausgehend von Fluorpivaloylcyanid, in 80%-iger Ausbeute zunächst rohes 4-Amino-6-fluor-tert.-butyl-3-mercapto-1,2,4-triazin-5-on und durch weitere Umsetzung mit Methyljodid ebenfalls in 80%-iger Ausbeute 4-Amino-6-fluor-tert-butyl-3-methylthio-1,2,4-triazin-5-on vom Schmelzpunkt 121-122°C.

In analoger Weise und entsprechend dem erfindungsgemäßen Verfahren werden die in der nachfolgenden Tabelle 1 aufgeführten Verbindungen der allgemeinen Formel (I)

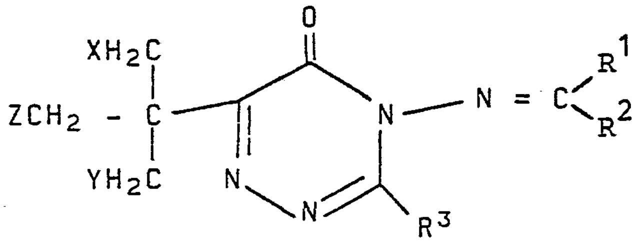

(I)

erhalten:

Tabelle 1

| Beispiel Nr. | X | Y | Z | $R^1$ | $R^2$ | $R^3$ | Schmelzpunkt (°C) |
|---|---|---|---|---|---|---|---|
| 3 | F | H | H | H | 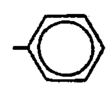 | $-SCH_3$ | 134-37 |
| 4 | F | H | H | $CH_3$ | $-CH_3$ | $-SCH_3$ | 106-10 |
| 5 | Cl | H | H | H | $-CH(CH_3)_2$ | $-SCH_3$ | 83-90 |
| 6 | Cl | H | H | H | 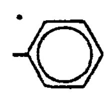 | $-SCH_3$ | 125-27 |
| 7 | F | F | H | H | $-CH(CH_3)_2$ | $-SCH_3$ | 110-13 |
| 8 | F | H | H | H | 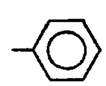 | $-N(CH_3)_2$ | 100-03 |
| 9 | F | H | H | $CH_3$ | $-CH_3$ | $-N(CH_3)_2$ | 76-80 |
| 10 | F | H | H | H | $-CH(CH_3)_2$ | $-N(CH_3)_2$ | 81-83 |

| Beispiel Nr. | X | Y | Z | $R^1$ | $R^2$ | $R^3$ | Schmelzpunkt (°C) |
|---|---|---|---|---|---|---|---|
| 11 | Cl | H | H | $CH_3$ | $-CH_3$ | $-NHCH_3$ | 168-170 |
| 12 | Cl | H | H | H | $-CH(CH_3)_2$ | $-NHCH_3$ | 119-120 |
| 13 | Cl | H | H | $CH_3$ | $-CH_3$ | $-N(CH_3)_2$ | 70 |
| 14 | Cl | H | H | H | $-CH(CH_3)_2$ | $-N(CH_3)_2$ | 59-60 |
| 15 | Cl | H | H | H | 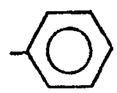 | $-N(CH_3)_2$ | 113-115 |

Verwendungsbeispiele

In den nachfolgenden Beispielen wird die nachstehend angegebene Verbindung als Vergleichssubstanz eingesetzt:

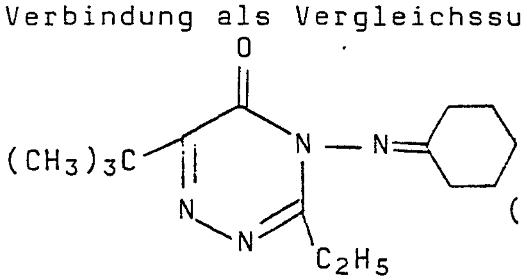

(A)

3-Ethyl-4-cyclohexylidenamino-6-tert.-butyl-1,2,4-triazin-5-on (bekannt aus DE-OS 22 38 206).

Beispiel A

Pre-emergence-Test

Lösungsmittel: 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in derZubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen z.B. die Verbindungen gemäß Herstellungsbeispielen 2,4,5 und 7 neben einer besseren allgemeinen herbiziden Wirksamkeit insbesondere eine bessere Selektivität in Mais, Sojabohnen und Baumwolle als die aus dem Stand der Technik bekannte Verbindung (A).

0074539

Patentansprüche

1) Azomethine von 4-Amino-6-halogen-tert.-butyl-1,2,4-triazin-5-onen der allgemeinen Formel I

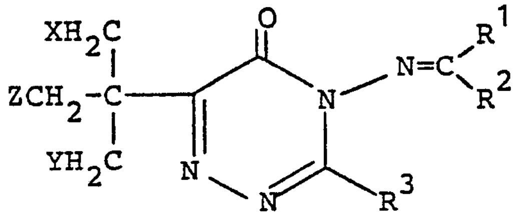

(I)

in welcher

$R^1$ für Wasserstoff oder Alkyl steht,

$R^2$ für Alkyl, Cycloalkyl, Cycloalkenyl oder für gegebenenfalls substituiertes Phenyl steht,

$R^3$ für Alkylthio, Alkylamino oder Dialkylamino steht,

X für Halogen steht und

Y und Z für Wasserstoff oder Halogen stehen.

2) Azomethine der allgemeinen Formel (I) gemäß Anspruch 1, dadurch gekennzeichnet, daß

$R^1$ für Wasserstoff oder Alkyl mit 1 bis 6 Kohlenstoffatomen steht,

$R^2$ für Alkyl mit 1 bis 6 Kohlenstoffatomen, Cycloalkyl mit 3 bis 6 Kohlenstoffatomen, Cycloalkenyl mit 5 bis 6 Kohlenstoffatomen oder für Phenyl, welches

gegebenenfalls durch Halogen, Alkyl mit 1 bis 4 Kohlenstoffatomen, Halogenalkyl mit 1 bis 2 Kohlenstoff- und bis zu 5 gleichen oder verschiedenen Halogenatomen, Alkoxy oder Alkylthio mit jeweils 1 bis 2 Kohlenstoffatomen, Cyano oder Nitro substituiert sein kann, steht,

$R^3$ für Alkylthio mit 1 bis 4 Kohlenstoffatomen oder für Alkylamino oder Dialkylamino mit jeweils 1 bis 6 Kohlenstoffatomen in jedem Alkylteil steht,

X für Halogen steht und

Y und Z für Wasserstoff oder Halogen stehen.

3) 6-Fluor-tert.-butyl-4-isobutylidenamino-3-methylthio-1,2,4-triazin-5-on der Formel

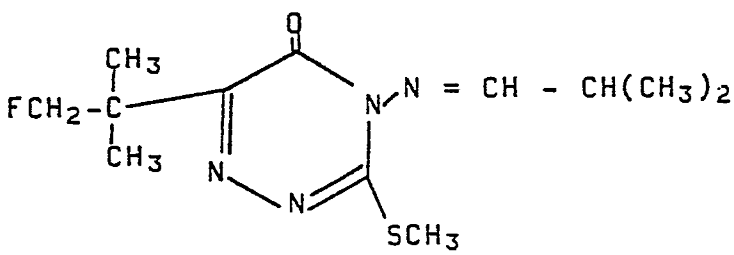

(2)

gemäß Anspruch 1.

4) 6-Fluor-tert.-butyl-4-isopropylidenamino-3-methylthio-1,2,4-triazin-5-on der Formel

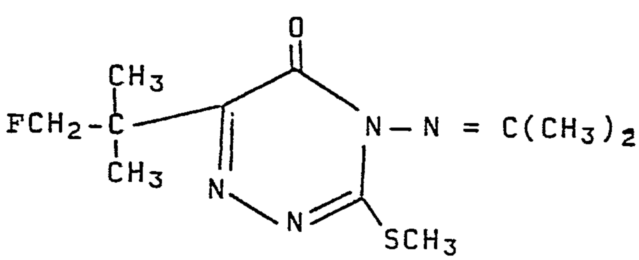

(4)

gemäß Anspruch 1.

5) 6-Chlor-tert.-butyl-4-isobutylidenamino-3-methylthio-1,2,4-triazin-5-on der Formel

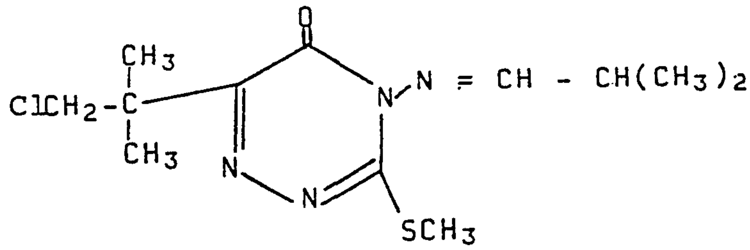

(5)

gemäß Anspruch 1.

6) 6-/1,1-Bis-(fluormethyl)-ethyl/-4-isobutylidenamino-3-methylthio-1,2,4-triazin-5-on der Formel

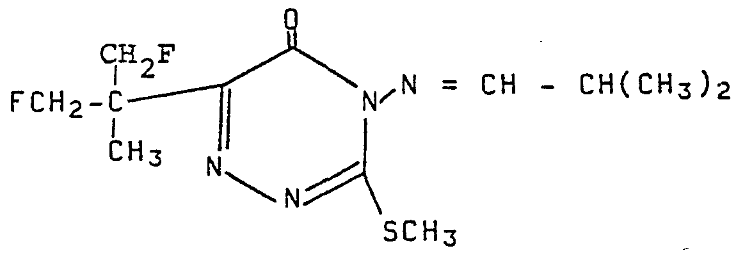

(7)

gemäß Anspruch 1.

7) Verfahren zur Herstellung von Azomethinen von 4-Amino-6-halogen-tert.-butyl-1,2,4-triazin-5-onen der allgemeinen Formel I

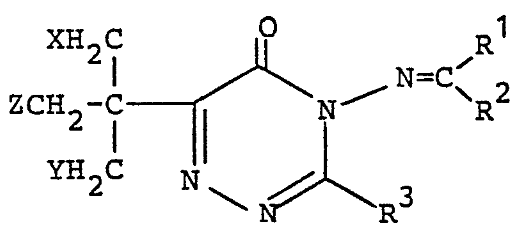

(I)

in welcher

$R^1$ für Wasserstoff oder Alkyl steht,

$R^2$ für Alkyl, Cycloalkyl, Cycloalkenyl oder für gegebenenfalls substituiertes Phenyl steht,

$R^3$ für Alkylthio, Alkylamino oder Dialkylamino steht,

X für Halogen steht und

Y und Z für Wasserstoff oder Halogen stehen,

dadurch gekennzeichnet, daß man 4-Amino-6-halogen-tert.-butyl-1,2,4-triazin-5-one der allgemeinen Formel II

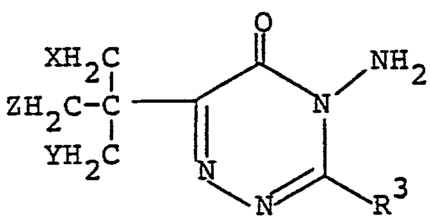

(II)

in welcher
$R^3$, X, Y und Z die oben angegebene Bedeutung haben,

mit Carbonylverbindungen der Formel III

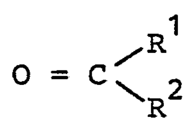

(III)

in welcher
$R^1$ und $R^2$ die oben angegebene Bedeutung haben,

gegebenenfalls in Gegenwart eines Verdünnungsmittels und gegebenenfalls in Gegenwart eines basischen oder sauren Katalysators umsetzt, wobei die Carbonylverbindungen der Formel (III) gegebenenfalls auch in Form von Acetalen bzw. Ketalen eingesetzt werden können.

8) Herbizide Mittel, gekennzeichnet durch einen Gehalt an mindestens einem Azomethin von 4-Amino-6-halogen-tert.-butyl-1,2,4-triazin-5-onen der Formel (I) gemäß Anspruch 1 bzw. 7.

9) Verwendung von Azomethinen von 4-Amino-6-halogen-tert.-butyl-1,2,4-triazin-5-onen der Formel (I) gemäß Anspruch 1 bzw. 7 zur Bekämpfung von unerwünschtem Pflanzenwachstum.

10) Verfahren zur Herstellung von herbiziden Mitteln, dadurch gekennzeichnet, daß man Azomethine von 4-Amino-6-halogen-tert.-butyl-1,2,4-triazin-5-one der Formel (I) gemäß Anspruch 1 bzw. 7 mit Streckmitteln und/oder oberflächenaktiven Mitteln vermischt.

Europäisches Patentamt

# EUROPÄISCHER RECHERCHENBERICHT

0074539
Nummer der Anmeldung

EP 82 10 7875

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-3 671 523 (K.WESTPHAL)<br>*Insgesamt; besonder Spalte 1, Zeile 45* | 1-2,7-10 | C 07 D 253/06<br>A 01 N 43/64 //<br>C 07 C 121/34 |
| Y | DE-A-1 795 784 (BAYER)<br>*Insgesamt* | 1-2,7-10 | |
| A | DE-A-2 726 016 (CIBA-GEIGY) | 1-2,8-10 | |
| A | US-A-4 058 526 (W.MERZ)<br>*Patentansprüche* | 1-2 | |
| P,Y | EP-A-0 049 416 (BAYER)<br>*Insgesamt* | 1-2,8-10 | **RECHERCHIERTE SACHGEBIETE (Int. Cl. 3)**<br>C 07 D 253/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 08-12-1982 | NUYTS A.M.K.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument